# EUROPÄISCHE PATENTSCHRIFT

(11) **EP 1 596 728 B1**
(45) Veröffentlichungstag und Bekanntmachung des Hinweises auf die Patenterteilung: **23.08.2006**
(21) Anmeldenummer: 04709139.2
(22) Anmeldetag: 07.02.2004
(51) Int. Cl.: A61B 17/12

(54) **VORRICHTUNG ZUR VERWENDUNG BEI HEILPROZESSEN**
DEVICE TO BE USED IN HEALING PROCESSES
DISPOSITIF DESTINE A ETRE EMPLOYE DANS DES PROCESSUS DE GUERISON

(30) Priorität: 10.02.2003 DE 10305553
(43) Veröffentlichungstag der Anmeldung: 23.11.2005
(73) Patentinhaber: Microcuff GmbH, 69469 Weinheim (DE)
(72) Erfinder: GÖBEL, Lothar, 97070 Würzburg (DE)
(74) Vertreter: Küchler, Stefan
(86) Internationale Anmeldenummer: PCT/EP2004/001139
(87) Internationale Veröffentlichungsnummer: WO 2004/069057

(56) Entgegenhaltungen:
- EP-A- 1 097 675
- DE-A- 19 508 129
- US-A- 5 643 178
- US-A- 5 667 479

## Beschreibung

### Technisches Gebiet

Die Erfindung befasst sich mit einer Vorrichtung zur Verwendung bei Heilprozessen.

In der Medizintechnik sind Vorrichtungen bekannt, die zur Tamponade von Hohlräumen dienen. Die Vorrichtungen bestehen aus inflatierbaren elastischen Hohlkörpern. Verschiedene Größen dieser Hohlkörper sind bekannt, um damit Ostien unterschiedlicher Größe abdichten zu können. Verwendet werden auch Vorrichtungen die in ihrer äußeren Kontur so gestaltet sind, dass sie aufgeblasen einen Hohlraum voll ausfüllen können.

Bei der Tamponade, insbesondere von Räumen im Bereich biologischer Gewebe, besteht das Problem, dass die Tamponade gegebenenfalls nicht vollständig an die Kontur des Hohlraums angepasst ist und auf angrenzende Schleimhäute unerwünschte Drücke ausübt. Dieses Problem wird dadurch verschärft, dass die Tamponierblase nicht residualvolumig gestaltet ist und hohe Rückstellkräfte bei dem verwendeten Wandmaterial bestehen. Bei einer Tamponade von Nasenhöhlen besteht darüber hinaus noch das Problem, dass die Nasenhöhle ein streng zentralgesteuertes lokal nicht beeinflussbares Schwellkörpersystem aufweist, welches periodisch zirkadiane Druckschwankungen aufweist, welche sich zu dem Binnendruck einer nichtresidualvolumigen Tamponierblase addieren, wodurch die Gefahr verstärkt wird, dass durch die Tamponade die Gefäßperfusion des angrenzenden Gewebes unterbunden wird. Im Hinblick auf die sehr unterschiedlichen Größenverhältnisse bei Nasennebenhöhlen und der großen Variationsbreite der interindividuellen räumlichen Ausformung und Volumina im Bereich anatomischer Räume, wird eine Vielzahl von anatomisch vorgeformten Vorrichtungen benötigt. Dieses ist sehr kostenaufwendig.

Neben den genannten Vorrichtungen zur Tamponade von Ostien beziehungsweise Hohlräumen, werden in der Medizintechnik Katheter verwendet, die aus einem elastischen Katheterschaft und einem daran angebrachten auffüllbaren Ballonelement bestehen. Der Katheterschaft verfügt über einen Auffüllkanal der über eine Öffnung in der Katheterwand in das Balloninnere mündet. Das Ballonelement selbst, dient vorrangig zur sicheren mechanischen Verankerung des Katheters. Außerdem hat er häufig eine dichtende Funktion und verhindert beispielsweise dass Urin aus der Harnblase am Katheter vorbei durch die Hamröhre abfließt. Der am Katheter befestigte Ballon ist bestrebt, beim Füllen mit einem Fluid eine Kugelform anzunehmen. Dadurch überschreitet der größte Querschnitt des Ballons den Querschnitt des Ostiums des Hohlraumes und verhindert damit die Retraktion durch Anschmiegen an dem Rand der Öffnung des Hohlraumes. Die Kugelform des Ballons kann die Halte- und Dichtfunktion nur ungenügend erfüllen, da sie unter Zugbelastung die Tendenz hat, eine spindelförmige Form anzunehmen und in das Ostium zu schlupfen, wodurch die Fixierung und die relativ kleine dichtende Kontaktfläche zwischen Ballonwand und Rand des Hohlraumostiums verloren geht. Dieses ist ein Problem, das im Bereich biologischer Gewebe besondere Bedeutung hat, da die Ostien der Körperhöhlen für gewöhnlich keine fixe Weite aufweisen. Aus diesem Grund auch, werden mehr oder weniger großflächige Rückhaltescheiben aus starrem Material an dem Katheterschaft angebracht, die aufgrund ihrer voluminösen Bauweise bei kleinen Ostien, die in dem Bereich von Millimetern liegen, nicht verwendbar sind. Darüber hinaus ist der kugelförmige Ballon auf den durchgehenden Trägerkörper, das heißt den Katheterschaft, angewiesen, der insbesondere bei engen Räumen sehr störend wirken kann.

### Stand der Technik

Durch die EP 0 624 349 B1 ist eine Vorrichtung zur Tamponade und zum Offenhalten von knochenbegrenzten Körperhöhlen und Gängen nach chirurgischer Manipulation bekannt, bei der die Außenkontur des Ballons im mit Fluid gefüllten Zustand der Innenkontur der Körperhöhle angepasst ist. Bei dieser Vorrichtung ist der Ballon als anatomisch idealisiert geformter Katheter ausgebildet und an die menschliche Stirnhöhle oder Siebbeinhöhle keilförmig angepasst. Der Nachteil dieser Vorrichtung besteht vor allem darin, dass eine Vielzahl von Größen bei einer großen Variationsbreite der Ausformung dieser Räume erforderlich ist.

### Darstellung der Erfindung

Der Erfindung liegt die Aufgabe zu Grunde, eine Vorrichtung zur Verwendung bei Heilprozessen zu schaffen, welche die oben angeführten Nachteile vermeidet und vielseitig einsetzbar ist. Die Vorrichtung soll möglichst sowohl bei der Tamponierung als auch bei der Anbringung von Kathetern verwendbar sein. Schließlich soll sie möglichst kostengünstig hergestellt werden können und mit Bezug auf die natürlich gegebenen Größen der Hohlräume in beiden Bereichen eingesetzt werden können.

Die Lösung der gestellten Aufgabe erfolgt erfindungsgemäß durch die Merkmale des Anspruchs 1. Die Unteransprüche 2 bis 27 geben vorteilhafte Weiterbildungen des im Anspruch 1 enthaltenen Erfindungsgedankens wieder.

Die Ausbildung der Vorrichtung als flexibles doppelwandiges inflatierbares Schlauchsegment, gibt die Möglichkeit eines weiten Anwendungsgebietes. Darüber hinaus ist die Vorrichtung sehr einfach herstellbar.

In der einfachsten Ausführungsform wird das Schlauchsegment durch eine Innenwand und eine Außenwand gebildet, die einen Hohlraum umschließen, wobei mindestens die Außenwand dünnwandig und elastisch dehnbar ist. Bei der Einführung eines Fluids, das heißt, einer Flüssigkeit oder eines Gases in das Schlauchsegment wird die Außenwand des Schlauchsegmentes aufgefaltet und legt sich dabei an die Wände des Ostiums oder an die Wände eines aufzufüllenden Hohlraumes an. Die Auffaltung und die elastische Dehnbarkeit der Außenwand dient dabei der vollständigen Anpassung der Außenwand an die räumlichen Gegebenheiten.

Von Vorteil ist, wenn das Schlauchsegment aus einem transparenten Material besteht. Als besonders geeignetes Material kommen hier Polyurethan, oder eine Polyurethan-Polyvinyilfloriertem-Mischung oder einem vergleichbaren Material auf Polyurethanbasis oder einem Polymer vergleichbaren Dehnungs- und Verarbeitungscharakteristika in Frage. Bei einer Verwendung diesen Materials kann das Schlauchsegment besonders dünnwandig hergestellt werden. Die anzustrebende Wandstärke liegt im Mikrometerbereich und zwar vorzugsweise bei 5 bis 15 Mikrometer. Außerdem kann eine Sonde von außen in das Schlauchsegment eingeführt und der Hohlraum von innen betrachtet werden. Ein solches Schlauchsegment kann sowohl als Tamponade von Hohlräumen oder Ostien als auch zur reversiblen, dichtenden Fixierung von Kathetern verwendet werden, in dem es am Ende eines Katheters angeordnet ist.

Die Vorrichtung ist aufgrund ihrer Eigenschaften besonders gut bei der Tamponade von natürlichen oder künstlich angelegten Ostien geeignet. Auch Katheter können in Hohlorganen wie Harnblase, Magen oder Darm gut fixiert werden. Durch die neue Fixierung resultiert auch eine bessere Dichtung gegen die Öffnung des Hohlraumes als das bei einem kugelförmigen Ballon möglich wäre, da ein dichtender Kontakt nicht mit einer relativ kleinen Fläche der Hohlraumwand unmittelbar benachbart zum Ostium des Hohlraumes stattfindet, sondern mit einer weit größeren Kontaktfläche, die durch den durch das Schlauchsegment gegebenen, proximalen Toruswulst zustande kommt. Beim Füllen des inflatierbaren Schlauchsegmentes mit dem Fluid, entsteht ein längs gestreckter Torus mit besonders günstigen Dichtungseigenschaften.

Die Herstellung des Schlauchsegmentes erfolgt in besonders günstiger Weise durch eine Invagination eines einwandigen Schlauchabschnittes. Ein Schlauchabschnitt von vorgegebener Länge, beispielsweise von 10 cm wird ineinander gestülpt, so dass beide Enden des Schlauchabschnittes in etwa übereinander liegen. Die Enden können dann an ein Abschlusselement in der Form eines Rohrnippels oder auch an einer geeigneten Stelle eines Katheters befestigt werden. In den von den Wänden des so gebildeten Schlauchsegmentes entstandene Innenraum, wird ein Kanal für die Zubeziehungsweise Ableitung eines Fluids eingesetzt. Wird ein Fluid in den Innenraum des Schlauchsegmentes eingeführt, so faltet und dehnt sich die Außenwand des Schlauchsegmentes auf und kann an entsprechender Stelle eingesetzt zur Tamponierung beziehungsweise Fixierung eines Katheters dienen.

Um die besonders gute Wirkungsweise des Erfindungsgegenstandes erreichen zu können, wird das Schlauchsegment vor seiner Ausbildung durch Invagination als einwandiger Schlauch präformiert. Diese Präformierung wird bevorzugt so durchgeführt, dass der die Außenwand des Schlauchsegmentes bildende Schlauchanteil nach der Invagination in der Rotationsebene des Schlauchsegmentes aufgebläht einen Torus ergibt. Dabei kann die Präformierung unterschiedlich stark sein, das heißt, dass nach der Präformierung und Invagination die Außenwand des Schlauchsegmentes an dessen Innenwand mehr oder weniger gefaltet anliegt. Dabei ist es auch möglich, durch die Präformierung mindestens die an dem äußeren Ende des Schlauchabschnittes angrenzende nach der Inflation vorhandene Stirnwand verstärkt auszubilden, um dadurch eine verbesserte Dichtwirkung für Sonderfälle zu erreichen.

Ganz generell wird bei der Präformierung des einwandigen Schlauches so vorgegangen, dass der Teil des Schlauches, welcher nach der Invagination die Innenwand des Schlauchsegmentes bildet, einen kleineren Querschnitt und eine größere Wandstärke aufweist als der nach der Invagination die Außenwand bildende Schlaüchanteil.

Ganz generell ist auch vorgesehen, dass die Vorrichtung bezüglich des Volumens einer von ihr zu tamponierenden Körperhöhle residualvolumig gestaltet ist, das heißt, dass das Schlauchsegment im frei entfalteten Zustand ein größeres Volumen aufweist als die zu tamponierende Körperhöhle.

Die Wandstärke, mindestens der Außenwand des Schlauchsegmentes, liegt im Bereich von wenigen Mikrometern, so dass eine gute Entfaltung der Außenwand möglich ist. Die bei einer Entfaltung in einem durch die Vorrichtung zu tamponierenden Hohlraum aus überschüssigem Wandmaterial entstehenden Falten liegen in der Dimension einer Kapillare. Hierdurch werden Flüssigkeiten aufgrund von Adhäsionskräften darin festgehalten.

Der in den Innenraum des Schlauchsegmentes mündende Kanal wird über einen flexiblen Verbindungsschlauch mit einem außerhalb des Schlauchsegmentes gelegenen Ventil verbunden. Das Ventil kann als Lippenventil ausgebildet sein. Möglich ist es aber auch, den Kanal mit einer zirkulären Manschette aus flexiblem Material zu versehen, durch die ein Rückströmen des Fluids aus dem Innenraum des Schlauchsegmentes verhindert wird.

Bei einigen Anwendungsgebieten kann es von Vorteil sein, wenn die Außenwand des Schlauchsegmentes aus einem polaren, in geringem Maße wasserdurchlässigem Material gebildet ist. Dieses Material kann beispielsweise eine semipermeable Membran sein.

Die Gestaltung der Vorrichtung als Schlauchsegment lässt es auch zu, dass zur Messung des transmuralen Druckes bei Inflation im Innenraum des Schlauchsegmentes ein Drucksensor angebracht ist. Dadurch lassen überhöhte Drücke bei der Inflation der Vorrichtung erkennen und vermeiden.

Damit das Fluid nicht ungewollt aus dem Innenraum des Schlauchsegments entweicht, ist im Kanal an geeigneter Stelle ein Ventil angebracht. Hier sind verschiedene Bauformen bezüglich der Anbringungsstelle im Kanal als auch bezüglich der konstruktiven Ausbildung des Ventils selbst möglich.

Gemäß einer Variante kann auf das Schlauchsegment ein Klemmverschluss aufgeschoben werden, der zum teilweisen oder auch vollständigen Abschluss des Kanals eine längs verschiebbare Manschette hat. Diese Manschette kann gleichzeitig durch Verschieben auch die Größe des Schlauchsegments selbst vorgeben.

Schließlich kann auf dem Rohrnippel bzw. dem Katheterschaft ein kragenförmiges Widerlager angebracht sein, welches ein Einklemmen und Fixieren einer Hohlraumwand am Schlauchsegment ermöglicht.

Das Schlauchsegment wird auf Grund der idealen Torusgeomentrie, die es bei der Inflation anstrebt, den gewollten Fixier- und Dichtanforderungen gerecht. Das Schlauchsegment kann in deflatiertem Zustand derart längs ausgestreckt zusammengefaltet liegen, dass es durch sehr enge Öffnungen eingeführt werden kann. Als Positionierhilfe kann das Schlauchsegment bei Bedarf mit einem Führungsstab oder einem Führungsröhrchen ausgestattet sein. Der ausgestreckte Doppelschlauchkörper weist jedoch selbst durch das enge Aneinanderliegen von vier Wandschichten im deflatierten Zustand eine gewisse Steifheit auf, die es allein bereits möglich macht, ihn für die meisten Anwendungen zu positionieren. Dieser Selbsttrageeffekt kann dadurch gesteigert werden, dass der invaginierte Schlauchwandteil wandstärker ausgebildet ist als der übrige Teil des Schlauchsegments.

Bei Inflation der proximal bzw. der distal zusammengefassten Schlauchenden längs des ausgestreckten Schlauchsegments entsteht eine Relativbewegung der Schlauchwandenden und des Schlauchkörpers. Es kommt dabei bei fortgesetzter Inflation zu einer Schlauchwandmaterialverschiebung zwischen dem rotationsachsennahen und dem rotationsachsenfernen Schlauchanteil, die ab dem Moment einsetzt, an dem sich die beiden der Rotationsachse zugewandten Schlauchpartien berühren, und die unterhalten wird, bis sich die zusammengefassten Schlauchwandenden dem Rotationszentrum des sich entfalteten Torus maximal angenähert haben und damit die energetisch günstigste Geometrie für das inflatierte Schlauchsegment eingenommen ist.

Sind dabei die beiden zusammengefassten Enden des Schlauches außerhalb der Hohlkörperwand fixiert, schmiegt sich das Schlauchsegment mit seiner ringförmigen Wulst an die Wand des Hohlraums an und drückt die Wand an das kragenförmige Widerlager an. Das Schlauchsegment wird in seiner Position gehalten, solange der Binnendruck aufrecht erhalten wird.

Ist das Schlauchsegment in deflatiertem Zustand proximal ausgestreckt, so resultiert bei Inflation eine Bewegung des Schlauchsegments nach distal, die geeignet sit, das Schlauchsegment in einen Hohlraum hinein zu entfalten. Das Schlauchsegment ist dadurch auch geeignet, Stoffe aus einem Hohlraum zu verdrängen.

Das Schlauchsegment eignet sich auf Grund seiner dünnwandigen und residualvolumigen Gestaltung, die ohne durchgängigen Trägerkörper auskommt, besonders zur Tamponade strukturell komplizierter Räume bzw. von Räumen mit einer druckempfindlichen Schleimhaut, wie z.B. der Nasenhöhle und deren Nebenhöhlen. Es wird dadurch allgemeinen Anwendungen gerecht, bei denen eine direkte Übertragung des Binnendruckes ohne Addition von Retraktionskraft des Wandmaterials des Schlauchsegments auf die Körperhöhlenwand nötig ist, um auf diese Weise durch ein an den Kanal extern angeschlossenes Manometer den unmittelbar auf das umgebende Gewebe wirkenden Druck messen zu können, mit dem Ziel, durch die Tamponade den Gefäßperfusionsdruck des angrenzenden Gewebes nicht zu überschreiten. Die Torusform des Schlauchsegments bietet auch noch die Möglichkeit, einen Drucksensor, ohne dass er an der Grenzschicht zum umgebenden Gewebe störend wirkt, im Innenraum zwischen den rotationsachsennahen Schlauchanteilen des Doppelschlauchkörpers zu plazieren. Der hier gemessene Druck entspricht bei Residualvolumigkeit des Schlauchsegments dem Druck der über die achsenfernen Schlauchanteile auf das umgebende Gewebe übertragen wird.

Ist das Schlauchsegment mit einer dünnen Wandung aus Polyurethan, durch welche sich Wasser in kleinen Mengen "hindurchlöst", ausgerüstet, so kann das Schlauchsegment auch zur Drainage von Hohlräumen verwendet werden oder auch zur protrahierten Applikationen von polaren Medikamentenwirkstoffen, wie zum Beispiel N₂O, durch die Wandung hindurch von innen nach außen. Eine Nutzung solcher Wirkungen in umgekehrter Richtung ist natürlich gleichfalls möglich.

Die erfindungsgemäße Kombination von residualer Ballonbemaßung, mikrodünnwandiger Ausführung der Ballonhülle und schaft- bzw. katheterlosem Tamponadeschlauch macht die erfindungsgemäße Vorrichtung ebenfalls geeignet zum durchblutungsverträglichen, nicht perfusionsunterbindenden Einbringen strahlender Medien, zum Beispiel zur Verödung proliferierenden Gewebes im Rahmen chronisch entzündlicher Prozesse oder bei der präoperativen Tumorreduktion. Der Tamponadekörper gestattet die vollständige gewebeverträgliche Füllung auch komplex geformter knöcherner Hohlräume, wie zum Beispiel der Nasennebenhöhlen, mit einem strahlenden Medium, wobei die ausgeübte transmurale Kraft über alle Flächen der Cavität annäherd homogen ist, zu keiner relevanten Beeinträchtigung der Gewebeperfusion führt und das Medium im Anschluß aus der Cavität komfortabel und vollständig entfernt werden kann. Während bisher derartige Medien frei in den Körperhohlraum eingegeben werden und in der Regel nach Therapie nicht vollständig zurückgewonnen werden können, gestattet die vorliegende Schlauchtamponade somit eine vollständige Entfernung des Strahlers, in dem die Tamponade abgesaugt und der gesamte Körper der Tamponade anschließend einfach zurückgezogen wird. Der nuklearmedizinische Einsatz ist darüber hinaus denkbar bei der langfristigen perfusionsverträglichen Bestrahlung von Tumorgewebe in Hirn, Brust, Darm, intraabdominalen und intrathorakalen Organen, und natürlichen sowie chirurgisch eröffneten oder angelegten Körperräumen. Zur Vermeidung ungewollter Exposition von Gewebe außerhalb des zu therapierenden Areals kann die Tamponade durch entsprechende partielle Ummantelung bzw. durch Abschirmung mit einem für die Strahlung undurchlässigen Material geschützt werden. Das Material kann ein Metall sein und als separate Schicht oder als unmittelbarer Bestandteil des Schlauchsegmentes zur Anwendung gelangen, beispielsweise in Gestalt einer darauf aufgedampften, metallischen Schicht.

Neben der radiotherapeutischen, nuklearmedizinischen Nutzbarkeit der Schlauchtamponade ist ebenfalls eine radiodiagnostische Verwendung denkbar. Statt eines Strahlers kann röntgendichtes Kontrastmittel in den Tamponadekörper eingebracht werden um Körperhohlräume oder Organe in toto darzustellen und eine direkte Exposition des Gewebes mit der Substanz zu vermeiden bzw. die systemische Aufnahme der Substanz in den Organismus auszuschließen.

Ist das Schlauchsegment mit einem internen Ventilmechanismus ausgerüstet, so kann das Schlauchsegment nach Inflation von der Fluidzufuhr getrennt werden. Dies macht es möglich, das Schlauchsegment im inflatierten Zustand, z.B. im Einsatz zur Tamponade der Nasenhöhle, von störenden Zuleitungen zu trennen und das proximale Ende des Verschlusses, extern an der Körperoberfläche zu fixieren. Dazu kann der ganz oder teilweise retratierte, doppelt längsgeschlitzte und bei der Retraktion über dem Abschlusselement gespreizte Klemmverschluss dienen, wenn er in der Weise ausgeführt ist, dass er nicht auf der ganzen Länge geteilt vorliegt, sondern distal einen geschlossenen, das noch zusammengefaltete Schlauchwandmaterial umfassenden Rohranteil aufweist und proximal in einer beliebigen Position durch eine Manschette die Verschiebbarkeit gegen den darunter liegenden, nicht entfalteten, Schlauchanteil festgestellt werden kann.

Für den speziellen Fall der Nasentamponade nach einer Nasenseptumoperation kann das Schlauchsegment in der Weise genutzt werden, dass es in seiner zentralen, lichten Öffnung mit einer Klammer ausgestattet wird, die einseitig den entfaltet drehrunden Doppelschlauchköper zusammenrafft und so eine plane Anlagefläche im Sinne einer Schiene für das Nasenseptum möglich macht, wobei die Schienung durch die Tamponade des übrigen Raumes der Nasenhöhle mittels des nun kissenartigen, gegenüberliegenden Teils des Schlauchsegements unterhalten wird. Der dem Nasenseptum anliegende Schenkel der klammerförmig ausgebildeten Schiene kann dabei zusätzlich als Träger für Therapeutika dienen und zur Aufrechterhaltung der Raffung des Schlauchwandmaterials am Abschlusselement fixiert werden.

Die erfindungsgemäße Relativbewegung zwischen aufgeblasenem Tamponadeschlauch und dem schlauchfixierenden Rohrnippel macht die vorliegende Schlauchtamponade in besonderer Weise nutzbar für die Dichtung des Anus bei Patienten mit rektalem Inkontinenzsyndrom. Der ringförmige Wulst der sich beim Befüllen des Tamponadekörpers einstellt, schmiegt sich von innen an den raktalen Schließmuskel und sitzt diesem wie eine dichtende Kappe auf. Wird der die Schlauchenden fixierende Nippel außerhalb des Körpers plaziert und dort mit einem Widerlager verbunden, welches den fixierenden Nippel in der Analfalte hält und ein Hineingleiten des Nippels in den Anus bzw. das Rektum verhindert, führt die Gegenbewegung von entfaltetem Tamponadeschlauch und extrakorporalem Fixierelement zu einer pressenden Dichtung des Ballonkörpers auf dem Rektumboden und wirkt so der Inkontinenz entgegen.

Das Widerlager kann in Form eines ankerartigen, im wesentlichen im rechten Winkel zum Ballonkörper stehenden Schlauch- oder Stabelement ausgeführt werden oder als dem fixierenden Nippel aufsitzender, eigenständiger Ballon, der, als mögliche Ausführungsvariante, über das Füll-Lumen des Inneren Ballons mitversorgt wird. Über das freie, offene Lumen des in sich zurückgestülpten Tamponierballons sowie einen entprechend ausgeformten Fixiernippel kann wiederum ein drainierender oder zuleitender Katheter eingeführt werden.

Das Schlauchsegment kann auch dazu dienen, an seiner Oberfläche fixierte Substanzen oder Körper in unmittelbaren Berühungskontakt mit der Körperhöhlung zu bringen, um die therapeutischen Effekte auf die zu behandelnde Stelle zu focussieren. Es ist beispielsweise möglich, auf der Oberfläche des Schlauchsegmentes nach außen geführte Elektroden zu fixieren, um eine Stimulierung des Körpergewebes durch eine elektrische Spannung zu bewirken oder dort vorhandene Spannungen abzugreifen und zu messen. Die Elektroden bestehen regelmäßig aus Metall. Sie können aufgeklebt oder aufgedampft sein.

Ferner ist es möglich, auf dem Schlauchsegment Behälter oder Träger zu fixieren, die radioaktive oder chemotherapeutische Agentien enthalten. Derartige Behälter oder Träger können unmittelbar an die zu behandelnde Stelle angepresst werden, was die gezielte Behandlung erleichtert und es vereinfacht, unerwünschte Sekundärschäden an dem umgebenden, gesunden Gewebe zu vermeiden.

### Kurzbeschreibung der Zeichnung

Anhand mehrerer Ausführungsbeispiele wird die Erfindung nachstehend näher erläutert:

Es zeigen:
- Figur 1: schematisch einen präformierten Schlauchabschnitt,
- Figur 2: den präformierten Schlauchabschnitt nach Figur 1, durch Invagination ausgebildet zum Schlauchsegment,
- Figur 3: ein inflatiertes Schlauchsegment im Längsschnitt,
- Figur 4: einen Längsschnitt in schematischer Darstellung durch eine andere Ausbildungsform eines Schlauchsegmentes an einem Katheter,
- Figur 5: ein Schlauchsegment im Längsschnitt mit einer eingesetzten Klammer und
- Figur 6: ein Schlauchsegment mit einem aufgeschobenen Klemmverschluss.

### Ausführung der Erfindung

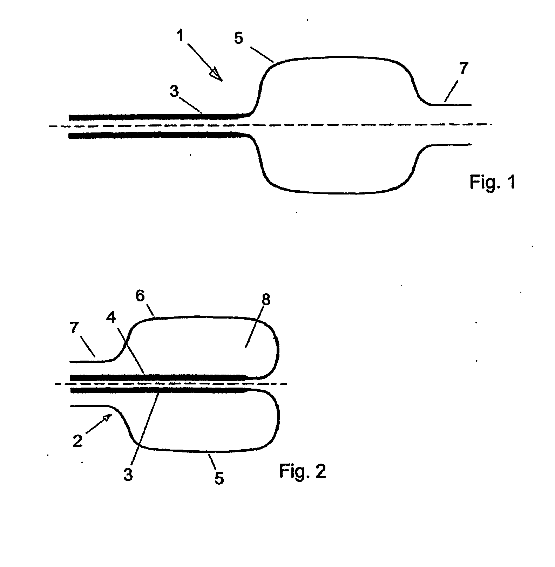

In der Figur 1 ist ein Schlauchabschnitt 1 gezeigt, welcher für die Herstellung eines Schlauchsegmentes 2 präformiert ist. Der Schlauchanteil 3, welcher die spätere Innenwand 4 des Schlauchsegmentes 2 bildet, ist, soweit es seine Wandstärke und seinen Innen- beziehungsweise Außendurchmesser betrifft, unverändert. Dagegen ist der Schlauchanteil 5, welcher die spätere Außenwand 6 des Schlauchsegmentes 2 bildet, erheblich aufgeweitet, wodurch die Wandstärke stark vermindert wurde. Auch das an diesen Schlauchanteil 5 anschließende Schlauchende 7 ist teilweise aufgeweitet. Diese Präformierung wird in beheizbaren Formeinrichtungen durchgeführt. Als Material für den Schlauchabschnitt 1 beziehungsweise Schlauchsegment 2, wird ein transparentes Polyurethan verwendet.

Zur Bildung des Schlauchsegmentes 2 wird das relativ stabile Schlauchteil 3 in den Innenraum des Schlauchanteils 5 eingedrückt und das Schlauchende 7 übergewälzt, so dass die in der Figur 2 gezeigte Form entsteht.

In der Figur 2 ist die aufgefaltete Form eines Schlauchsegmentes 2, eingezeichnet. Zu diesem Zweck ist ein Fluid in den Innenraum 8, welcher von der Innenwand 4 und der Außenwand 6 begrenzt ist, eingefüllt. Bei entleertem Innenraum liegt die Außenwand 6 in eingefaltetem Zustand an der Innenwand 4 an.

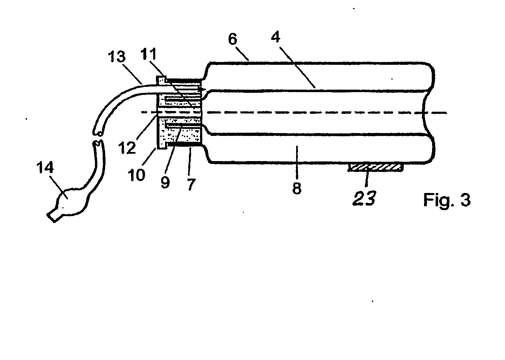

Die Figur 3 zeigt ein praktisches Ausführungsbeispiel des Schlauchsegmentes 2, wie es zur Tamponierung eingesetzt werden kann. Die beiden Enden 7 und 9 des Schlauchsegmentes 2, sind von dem Abschlusselement 10 fluiddicht erfasst. Das Abschlusselement 10 ist in der Form eines Rohrnippels ausgebildet. Die in der Mitte des Rohrnippels 10 vorhandene Öffnung 11 kann durch einen Stopfen 12 verschlossen werden. Möglich ist es aber auch, in die Öffnung 11 einen Katheterschaft einzuführen. Der Innenraum 8 des Schlauchsegmentes 2 ist an dem Kanal 13 angeschlossen, welcher für die Zubeziehungsweise Ableitung eines Fluids vorgesehen ist. Das Schlauchsegment 2 ist inflatiert und stark vergrößert dargestellt. In den Kanal 13 ist ein Ventil 14 eingesetzt, das einen ungewollten Abfluss des Fluids aus dem Innenraum 8 verhindert. Im Beispiel ist das Ventil 14 durch eine an sich bekanntes Lippenventil mit elastisch aneinander anliegenden Ventillippen gebildet.

Auf der Oberfläche des Schlauchsegmentes 2 ist lokal ein Körper 23 fixiert, der eine chemotheraperutische oder radioaktive Substanz enthält. Die Körper wird im aufgeblähten Zustand des Schlauchsegmentes mit der zu behandelnden Körperstelle verpresst, was es erlaubt, lokal eine besonders intensive Wirksamkeit zu entfalten unter Vermeidung von Schädigungen an dem umgebenden, gesunden Körpergewebe.

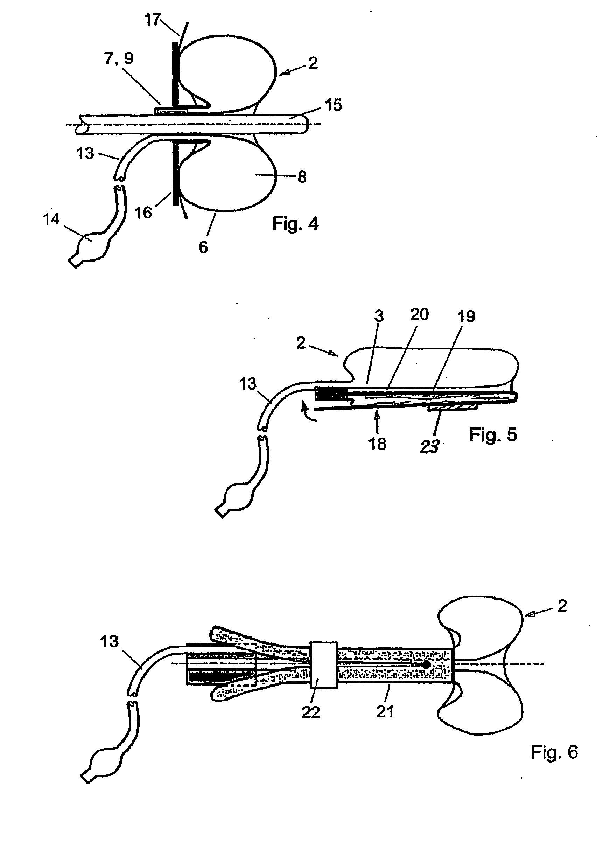

In der Figur 4 ist ein Ausführungsbeispiel gezeigt, bei dem das Schlauchsegment 2 am Ende eines Katheters 15 angebracht ist. Die Enden 7, 9 des Schlauchsegments 2 sind einander umschließend mit dem Katheterschlauch 15 verbunden. Der Kanal 13 führt in den Innenraum 8 des Schlauchsegments 2. Das Beispiel zeigt die Anbringung des Schlauchsegments 2 in einem nicht näher umrissenen Hohlraum. In diesem Fall kann auf dem Katheterschaft 15 ein ringförmiges Widerlager 16 kragenförmig aufgesetzt sein, wodurch beispielsweise die Haut 17 an der Öffnung des Hohlraums zwischen dem Widerlager 16 und der Außenwand 6 des Schlauchsegments 2 dichtend eingeklemmt werden kann. Eine solche Ausbildung erlaubt es, z.B. einen Körperhohlraum mit einer Flüssigkeit kontrolliert zu spühlen. Eine Verschmutzung der Umgebung ist durch das dichtend an der Haut anliegende, ringfömige Widerlager ausgeschlossen.

Die Figur 5 zeigt den Einsatz des Schlauchsegments 2 mit gleichzeitiger Anwendung einer Klammer 18. Die Klammer 18 besteht aus steifem Material und wird mit ihrem einen Schenkel 19 in den freien Raum 20 des Schlauchanteils 3 eingeschoben. Durch die Klammer 18 kann das Schlauchsegment 2 auf einer gewünschten Seite mit einem steifen Anteil versehen werden. Es kann auch dazu verwendet werden, an seiner Oberfläche fixierte Substanzen oder Körper 23 zu tragen und in gezielter Weise in einer Körperhöhle zu platzieren und zur chemischen oder theraperapeutischen Heilbehandlung zu verwenden. Dabei ist die sanfte Verpressung mit dem Körper durch das rückseitig angebrachte und während der Anwendung aufgeblähte Schlauchsegment von entscheidender Bedeutung für den erzielten Heilerfolg. Die richtige und präzise Positonierung des Körpers in dem Hohlraum ist besonders einfach.

Die Figur 6 zeigt eine Anwendungsform des Schlauchsegments 2, bei der auf das Schlauchsegment 2 ein Klemmverschluss 21 aufgeschoben ist. Durch ein Verschieben des Klemmverschlusses 21 längs des Schlauchsegments 2 kann die Größe des aufgeblähten Anteils des Schlauchsegments 2 bestimmt werden. Je weiter der Klemmverschluss 21 auf der Zeichnung gesehen nach links geschoben wird, um so größer wird der freigegebene Anteil des Schlauchsegments 2. Der Klemmverschluss 21 sit zur Feststellung mit einer ebenfalls längs verschiebbaren Manschette 22 versehen. Der Klemmverschluss 21 ist über nahezu seiner gesamten Länge geteilt und in seiner Wandstärke so gewählt, dass ein Verschieben der Manschette längs des Klemmverschlusses 21 zu einem stärkeren oder schwächeren Abschluss des Schlauchsegments 2 bzw. des Kanals 13 führt. Zur Abschirmung gegen strahlende Medien kann der Klemmverschluß 21 in entsprechender Ausführung wie in Figur 6 aus folienartigem, strahlungsabschirmendem Material bestehen, beispielsweise aus einem ein- oder beidseitig mit einem Metall bedampften, polymeren Werkstoff oder insgesamt aus Metall.

Das neue Schlauchsegment kann, wie die Beispiele zeigen, vielseitig angewendet werden. Es ermöglicht auch einen verbesserten Zugang für Sichtsonden, Druckmesser und dergleichen in das Innere von Hohlräumen. Das Schlauchsegment lässt es sogar zu, dass dem Hohlraum Flüssigkeits- oder Feststoffanteile entnommen werden können, ohne die Verwendung besonderer Instrumente, indem allein durch das Ziehen an der Innenwand 4 unter gleichzeitiger Abstützung der Außenwand 6 durch die innenliegende Auswölbung des Schlauchsegments 2 eine Art lippenförmiger Verschluss gebildet wird.

## Patentansprüche

1. Vorrichtung zur Tamponade von Körperhöhlen, bspw. auch zwecks sicherer mechanischer Verankerung eines Katheters, umfassend ein flexibles , durch einen Schlauchabschnitt (1) geformtes, Schlauchsegment (2) mit einer Innenwand (4) und einer Außenwand (6), die einen Hohlraum (8) umschließen, so dass das Schlauchsegment (2) inflatierbar ist, wobei das Schlauchsegment (2) ohne durchgängigen Trägerkörper sowie derart ausgebildet ist, dass bei fortgesetzter Inflation eine Schlauchwandmaterialverschiebung zwischen der Innenwand (4) und der Außenwand (6) des Schlauchsegments (2) möglich ist,
wobei
a) der Schlauchabschnit (1) zwei Enden (7,9) aufweist, die beide an dem selben Abschlußelement (10) befestigt sind, so dass beim Füllen des inflatierbaren Schlauchsegments (2) eine Torusgeometrie angestrebt wird; und
b) das Abschlußelement (10) die Form eines Rohrnippels hat, durch den die beiden Enden (7,9) des Schlauchabschnitts (1) fluiddicht zusammengefaßt werden.

2. Vorrichtung nach Anspruch 1, **dadurch gekennzeichnet, dass** mindestens die Außenwand (6) dünnwandig und elastisch dehnbar ist.

3. Vorrichtung nach einem der Ansprüche 1 oder 2, **dadurch gekennzeichnet, dass** mindestens die Außenwand (6) des Schlauchkörpersegmentes (2) eine Wandstärke von wenigen Mikrometern hat.

4. Vorrichtung nach einem der Ansprüche 1 bis 3, **dadurch gekennzeichnet, dass** das Schlauchsegment (2) aus einem Polyurethan, einer Polyurethan-Polyvinylchlorid-Mischung oder einem vergleichbaren Material auf Polyurethanbasis oder einem Polymer mit vergleichbaren Dehnungs- und Verarbeitungscharakteristika besteht.

5. Vorrichtung nach einem der Ansprüche 1 bis 4, **dadurch gekennzeichnet, dass** das Schlauchsegment (2) durch eine Invagination eines einwandigen Schlauchabschnittes (1) gebildet ist.

6. Vorrichtung nach einem der Ansprüche 1 bis 5, **dadurch gekennzeichnet, dass** der Schlauchabschnitt (1) oder ein Teil desselben vor seiner Ausbildung zu einem Schlauchsegment (2) durch Invagination als einwandiger Schlauch walzenartig präformiert ist.

7. Vorrichtung nach Anspruch 6, **dadurch gekennzeichnet, dass** durch die Präformierung eine Verstärkung des bei der Invagination senkrecht zur Rotationsebene des Schlauchsegmentes (2) entstehenden Wulstes erzeugt ist.

8. Vorrichtung nach Anspruch 6, **dadurch gekennzeichnet, dass** der Schlauchabschnitt (1) derart präformiert ist, dass der Schlauchanteil (3), der nach der Invagination die Innenwand des Schlauchsegmentes (2) bildet, im Querschnitt kleiner ist und eine größere Wandstärke aufweist als der die Außenwand (6) bildende Schlauchanteil (5).

9. Vorrichtung nach einem der Ansprüche 1 bis 8, **dadurch gekennzeichnet, dass** der Schlauchanteil (3) mit einer gleichmäßigen Wandstärke und einem gleichmäßigen Innendurchmesser ausgeführt ist.

10. Vorrichtung nach einem der Ansprüche 1 bis 9, **dadurch gekennzeichnet, dass** das Schlauchsegment (2) residualvolumig ausgebildet ist.

11. Vorrichtung nach einem der Ansprüche 1 bis 10, **dadurch gekennzeichnet, dass** das Schlauchsegment (2) zur reversiblen dichtenden Fixierung eines Katheters am Ende eines Katheterschaftes (15) angeordnet ist.

12. Vorrichtung nach einem der Ansprüche 1 bis 11, **dadurch gekennzeichnet, dass** mindestens ein Ende (9) des Schlauchabschnittes (1) an einem Katheterschaft (15) befestigt ist.

13. Vorrichtung nach einem der Ansprüche 1 bis 12, **dadurch gekennzeichnet, dass** in den von den Wänden (4,6) des Schlauchsegmentes (2) gebildeten Innenraum (8) ein Kanal (13) für die Zu- bzw. Ableitung eines Fluids mündet.

14. Vorrichtung nach einem der Ansprüche 1 bis 13, **dadurch gekennzeichnet, dass** der Kanal (13) über einen flexiblen Verbindungsschlauch mit einem außerhalb des Schlauchsegmentes (2) gelegenen Ventil (14) verbunden ist, welches bevorzugt als Ventillippe ausgebildet ist.

15. Vorrichtung nach einem der Ansprüche 1 bis 13, **dadurch gekennzeichnet, dass** als Ventil (14) eine zirkuläre Manschette aus flexiblem Material vorgesehen ist, die zwischen den Schlauchenden (7,9) angebracht ist.

16. Vorrichtung nach einem der Ansprüche 1 bis 15, **dadurch gekennzeichnet, dass** auf das Schlauchsegment (2) ein Klemmverschluß (21) aufgeschoben ist, der eine längsverschiebbare Manschette (22) hat.

17. Vorrichtung nach einem der Ansprüche 1 bis 16, **dadurch gekennzeichnet, dass** auf dem Rohrnippel (10) bzw. Katheterschaft (15) ein kragenförmiges Widerlager (16) angebracht ist.

18. Vorrichtung nach einem der Ansprüche 1 bis 17, **dadurch gekennzeichnet, dass** in dem Innenraum (20) ein Drucksensor enthalten ist.

19. Vorrichtung nach einem der Ansprüche 1 bis 18, **dadurch gekennzeichnet, dass** in den von dem Schlauchsegment (2) umschlossenen Innenraum (8) ein Röntgenkontrastmittel und/oder eine medizinisch wirksame Substanz einbringbar ist.

20. Vorrichtung nach Anspruch 19, **dadurch gekennzeichnet, dass** das Schlauchsegment (2) in zumindest einem Teilbereich von einer Abschirmung (21) bedeckt ist und dass die Abschirmung die medizinische Wirksamkeit der Substanz in dem abgeschirmten Bereich unterdrückt oder vermindert.

21. Vorrichtung nach einem der Ansprüche 1 bis 20, **dadurch gekennzeichnet, dass** das Schlauchsegment (2) an seiner Oberfläche fixierte Substanzen aufweist, die vorzugsweise in zumindest einem Behälter oder Träger enthalten sind, der mit dem Schlauchsegment verbunden ist, oder Körper, die vorzugsweise durch nach außen geführte Elektroden gebildet sind.

22. Vorrichtung nach einem der Ansprüche 19 bis 21, **dadurch gekennzeichnet, dass** die Substanzen durch radioaktive oder chemotherapeutische Agentien gebildet sind.

## Claims

1. An apparatus for the tamponade of body cavities, for example also for reliable mechanical anchoring of a catheter, comprising a flexible hose segment (2) formed by a hose portion (1) with an inner wall (4) and an outer wall (6), which enclose a hollow space (8) so that the hose segment (2) is inflatable, wherein the hose segment (2) is without a permeable support member and is so formed that with continued inflation a displacement of hose wall material is possible between the inner wall (4) and the outer wall (6) of the hose segment (2), wherein
a) the hose portion (1) has two ends (7,9), both of which are secured to the same closure element (10) so as to aim for a toroidal geometry when filling the inflatable hose segment (2), and
b) the closure element (10) is in the shape of a tube nipple, through which the two ends (7,9) of the hose portion (1) can be fluidtightly combined.

2. An apparatus according to Claim 1, **characterised in that** at least the outer wall (6) is thin-walled and is elastically expandable.

3. An apparatus according to either Claim 1 or Claim 2, **characterised in that** at least the outer wall (6) of the hose body segment (2) has a wall thickness of a few microns.

4. An apparatus according to any one of Claims 1 to 3, **characterised in that** the hose segment (2) consists of a polyurethane, a polyurethane polyvinyl chloride mixture or a comparable material based on polyurethane or a polymer with comparable expansion and processing characteristics.

5. An apparatus according to any one of Claims 1 to 4, **characterised in that** the hose segment (2) is formed by invagination of a single-walled hose portion (1).

6. An apparatus according to any one of Claims 1 to 5, **characterised in that**, before it is formed into a hose segment (2), the hose portion (1) or a part thereof is preformed by invagination as a single-walled hose.

7. An apparatus according to Claim 6, **characterised in that** the preforming brings about a reinforcement of the bead formed during the invagination perpendicular to the plane of rotation of the hose segment (2).

8. An apparatus according to Claim 6, **characterised in that** the hose portion (1) is preformed so that the hose component (3), which after invagination forms the inner wall of the hose segment (2), is smaller in cross-section and has a larger wall thickness than the hose component (5) forming the outer wall (6).

9. An apparatus according to any one of Claims 1 to 8, **characterised in that** the hose component (3) is formed with uniform wall thickness and uniform inner diameter.

10. An apparatus according to any one of Claims 1 to 9, **characterised in that** the hose segment (2) is formed so as to have residual volume.

11. An apparatus according to any one of Claims 1 to 10, **characterised in that** the hose segment (2) is arranged for the reversible fluidtight fixing of a catheter at the end of a catheter shaft (15).

12. An apparatus according to any one of Claims 1 to 11, **characterised in that** at least one end (9) of the hose portion (1) is fastened to a catheter shaft (15).

13. An apparatus according to any one of Claims 1 to 12, **characterised in that** a duct (13) for the inlet and outlet of a fluid opens into the inner chamber (8) formed by the walls (4,6) of the hose segment (2).

14. An apparatus according to any one of Claims 1 to 13, **characterised in that** the duct (13) is connected via a flexible connecting hose to a valve (14) which is disposed outside the hose segment (2) and which, preferably, is in the form of a valve lip.

15. An apparatus according to any one of Claims 1 to 13, **characterised in that** as the valve (14) a circular cuff of flexible material is used, which is mounted between the hose ends (7,9).

16. An apparatus according to any one of Claims 1 to 15, **characterised in that** a clip seal (21), which has a longitudinally displaceable cuff (22), is pushed on to the hose segment (2).

17. An apparatus according to any one of Claims 1 to 16, **characterised in that** a collar-like abutment (16) is mounted on the tube nipple (10) or catheter shaft (15).

18. An apparatus according to any one of Claims 1 to 17, **characterised in that** a pressure sensors is included in the inner chamber (20).

19. An apparatus according to any one of Claims 1 to 18, **characterised in that** a radiographic contrast medium and/or a medicinally active substance can be introduced into the inner chamber (8) enclosed by the hose segment (2).

20. An apparatus according to Claim 19, **characterised in that** in at least one section the hose segment (2) is covered by shielding (21), and **in that** the shielding reduces the medicinal activity of the substance in the shielded zone.

21. An apparatus according to any one of Claims 1 to 20, **characterised in that** the hose segment (2) has substances fixed on its surface, which are preferably contained in at least one container or support which is connected to the hose segment, or members which are preferably formed by outwardly directed electrodes.

22. An apparatus according to any one of Claims 19 to 21, **characterised in that** the substances are formed by radio-active or chemotherapeutic agents.

## Revendications

1. Dispositif pour remplir des cavités corporelles, par exemple également pour l'ancrage mécanique fiable d'un cathéter, comprenant un segment (2) de tube flexible, formé par un tronçon (1) de tube, comportant une paroi intérieure (4) et une paroi extérieure (6), qui entourent une cavité (8), de sorte que le segment de tube (2) soit gonflable, le segment de tube (2) étant réalisé sans corps support continu et de manière à permettre, lors d'un gonflage continu, un déplacement de la matière de la paroi du tube entre la paroi intérieure (4) et la paroi extérieure (6) du segment de tube (2), dans lequel
a) le tronçon de tube (1) présente deux extrémités (7, 9), fixées toutes deux au même élément de fermeture (10), de façon à obtenir une géométrie torique lors du remplissage du segment de tube (2) gonflable, et
b) l'élément de fermeture (10) a la forme d'un raccord de tube par lequel les deux extrémités (7, 9) du tronçon de tube (1) sont réunies de manière étanche.

2. Dispositif selon la revendication 1, **caractérisé en ce qu'**au moins la paroi extérieure (6) est mince et extensible de manière élastique.

3. Dispositif selon l'une des revendications 1 ou 2, **caractérisé en ce qu'**au moins la paroi extérieure (6) du segment de corps de tube (2) a une épaisseur de paroi de quelques micromètres.

4. Dispositif selon l'une des revendications 1 à 3, **caractérisé en ce que** le segment de tube (2) est en polyuréthane, un mélange de polyuréthane-chlorure de polyvinyle ou une matière comparable à base de polyuréthane ou un polymère doté de caractéristiques d'allongement et de transformation comparables.

5. Dispositif selon l'une des revendications 1 à 4, **caractérisé en ce que** le segment de tube (2) est formé par invagination d'un tronçon de tube (1) à paroi simple.

6. Dispositif selon l'une des revendications 1 à 5, **caractérisé en ce que** le tronçon de tube (1) ou une partie de celui-ci, avant sa conformation en segment de tube (2) par invagination, est préformé de manière cylindrique sous forme de tube à paroi simple.

7. Dispositif selon la revendication 6, **caractérisé en ce que** la préformation génère un renforcement du renflement formé lors de l'invagination perpendiculairement au plan de rotation du segment de tube (2).

8. Dispositif selon la revendication 6, **caractérisé en ce que** le tronçon de tube (1) est préformé de façon que la portion de tube (3) qui, après invagination, forme la paroi intérieure du segment de tube (2), présente une section transversale plus faible et une épaisseur de paroi plus forte que la portion de tube (5) formant la paroi extérieure (6).

9. Dispositif selon l'une des revendications 1 à 8, **caractérisé en ce que** la portion de tube (3) est réalisée avec une épaisseur de paroi uniforme et un diamètre intérieur uniforme.

10. Dispositif selon l'une des revendications 1 à 9, **caractérisé en ce que** le segment de tube (2) est configuré avec un volume résiduel.

11. Dispositif selon l'une des revendications 1 à 10, **caractérisé en ce que** le segment de tube (2) est disposé à l'extrémité d'une tige de cathéter (15) pour la fixation étanche réversible d'un cathéter.

12. Dispositif selon l'une des revendications 1 à 11, **caractérisé en ce qu'**au moins une extrémité (9) du tronçon de tube (1) est fixée à une tige de cathéter (15).

13. Dispositif selon l'une des revendications 1 à 12, **caractérisé en ce qu'**un canal (13) d'alimentation et d'évacuation d'un fluide débouche dans l'espace intérieur (8) formé par les parois (4, 6) du segment de tube (2).

14. Dispositif selon l'une des revendications 1 à 13, **caractérisé en ce que** le canal (13) est relié, via un flexible de raccordement, à une soupape (14) placée à l'extérieur du segment de tube (2), laquelle est réalisée de préférence sous forme de soupape à lèvres.

15. Dispositif selon l'une des revendications 1 à 13, **caractérisé en ce que**, à titre de soupape (14) est prévue une manchette circulaire en matériau flexible, disposée entre les extrémités (7, 9) du tube.

16. Dispositif selon l'une des revendications 1 à 15, **caractérisé en ce que**, sur le segment de tube (2), est enfilée une fermeture à serrage qui comporte une manchette (22) déplaçable longitudinalement.

17. Dispositif selon l'une des revendications 1 à 16, **caractérisé en ce qu'**un aboutement (16) en forme de collet est disposé sur le raccord de tube (10) et/ou la tige du cathéter (15).

18. Dispositif selon l'une des revendications 1 à 17, **caractérisé en ce que** l'espace intérieur (20) contient un capteur de pression.

19. Dispositif selon l'une des revendications 1 à 18, **caractérisé en ce qu'**un moyen de contraste à rayons X et/ou une substance médicalement active peut être introduit(e) dans l'espace intérieur (8) qu'entoure le segment de tube (2).

20. Dispositif selon la revendication 19, **caractérisé en ce que** le segment de tube (2) est recouvert d'une protection (21) au moins sur une zone partielle et **en ce que** la protection supprime ou réduit l'efficacité médicale de la substance dans la zone protégée.

21. Dispositif selon l'une des revendications 1 à 20, **caractérisé en ce que** le segment de tube (2) comporte des substances fixées sur sa surface, qui sont contenues, de préférence, dans au moins un réservoir ou support relié au segment de tube, ou des corps qui sont formés de préférence par des électrodes guidées vers l'extérieur.

22. Dispositif selon l'une des revendications 19 à 21, **caractérisé en ce que** les substances sont formées par des agents radioactifs ou chimio-thérapeutiques.
